# EUROPEAN PATENT APPLICATION

(11) **EP 4 181 156 A1**
(43) Date of publication of application: **17.05.2023**
(21) Application number: 21210990.4
(22) Date of filing: 29.11.2021
(51) Int. Cl.: G16H 50/20, G16H 50/30, G16H 50/70, G16H 10/60

(54) **CLASSIFICATION OF SHOCK TYPE OF A SUBJECT**

(30) Priority: 10.11.2021 US 202163277749 P
(71) Applicant: Koninklijke Philips N.V., 5656 AG Eindhoven (NL)
(72) Inventor: DONG, Junzi, Eindhoven (NL); CHANG, Yale, Eindhoven (NL)
(74) Representative: Philips Intellectual Property & Standards

(57) **Abstract**

A mechanism or model for shock type classification that is able to differentiate between different types of shock, e.g. among patients with suspected hemodynamic instability. Respective one-versus-rest models are used to generate a numeric value for each of a plurality of shock types, each numeric value indicating a predicted probability that a subject exhibits that particular shock type over other types. A classification process is then performed to select the most likely shock type based on the numeric values.

## Description

### FIELD OF THE INVENTION

The present invention relates to the field of medicine, and in particular, to the processing of medical data.

### BACKGROUND OF THE INVENTION

In the medical field, shock is a critical condition during which the body cannot maintain sufficient blood pressure and oxygen supply to vital organs. It is possible to categorize shock into a number of different types, including at least cardiogenic shock, hypovolemic shock, and septic shock. Other known types of shock include anaphylactic shock and neurogenic shock. Different types of shock are treated differently, sometimes with directly contradictory therapies. Thus, for a subject exhibiting shock, it is important to accurately identify the type of shock to aid in providing the most appropriate treatment plan.

An automated mechanism that is able to differentiate between different types of shocks would therefore be useful for providing guidance to inexperienced clinicians, allowing experienced clinicians to respond faster by making orders of ultrasound or echocardiography to reassess the subject's condition and/or designing teaching modalities for training new clinicians.

There is therefore a desire to provide a mechanism that is able to predict a type of shock exhibited by a subject.

### SUMMARY OF THE INVENTION

The invention is defined by the claims.

According to examples in accordance with an aspect of the invention, there is provided a computer-implemented method of predicting a probable type of shock, amongst a plurality of potential types of shock, exhibited by a subject.

The computer-implemented method comprises: obtaining subject data comprising one or more data elements, each representative of medical information of the subject; for each of the plurality of potential types of shock: processing the subject data, using a respective one-versus-rest model, to produce respective numeric values, wherein each respective numeric value indicates a likelihood of whether the subject is exhibiting a respective type of shock over any other type of shock in the plurality of potential types of shock; and processing the numeric values using a classification model to generate a predictive indicator indicating the most probable type of shock amongst the plurality of potential types of shock.

The proposed approach makes use of a plurality of one-versus-rest models that are designed for discriminating for particular types of shock (compared to other types of shock). Each one-versus-rest model processes subject data (i.e. medical data of a subject) in order to provide a numeric value as output. The numeric value indicates a likelihood that the subject is exhibiting a particular type of shock (over any other type of shock). These numeric values are then further processed to identify the most probable type of shock.

The proposed approach provides an accurate mechanism for predicting the most probably type of shock. Advantages for predicting a type of shock exhibited by a particular subject have been previously described, and in particular, facilitate improved and specific treatment of the subject with a reduced risk of an incorrect or inappropriate treatment being performed.

The classification model may be configured to: perform a logistic function on each numeric value to produce a numeric probability for each numeric value, each numeric probability indicating a probability on a predetermined numeric scale that the subject exhibits a respective type of shock; and generate a predictive indicator identifying the type of shock associated with the numeric probability having the greatest value.

This embodiment provides a simple and resource-effective mechanism for generating the predictive indicator, which can be employed in low-resource (e.g. low power or low processing capabilities) systems. The logistic function may be any suitable numeric function that can provide a numeric probability, such as a softmax function.

Optionally, the classification model comprises a machine-learning model trained to receive, as input, at least the numeric values and provide, as output, the predictive indicator. This embodiment provides a more accurate mechanism for generating the predictive indicator, e.g. compared to rule-based differential diagnosis. This is because the machine-learning model effectively acts as a super classifier for generating the predictive indicator, which has been identified as generate a more accurate prediction of the type of shock (using outputs of the one-versus-rest models).

In some examples, the classification model is configured to further receive, as input, additional subject information containing one or more additional data elements, each representative of medical information of the subject. Use of additional subject (i.e. patient) information yields more accurate predictions of the type of shock. In particular, information that may have not been taken into account by one or more of the one-versus-rest models (e.g. due to overfitting or undertraining) can be processed or used by the machine-learning model in order to generate the predictive indicator.

Preferably, the plurality of potential types of shock includes at least three types of shock. In some examples, the at least three types of shock comprises: cardiogenic shock, septic shock, and hypovolemic shock. The present invention is particularly advantageous when used with these three types of shock, as they all result in hemodynamic instability and form the dominant categories of shock. Thus, a hemodynamic analytics suite can be advantageously constructed that first predicts hemodynamic instability before identifying the type of shock.

In some examples, the plurality of potential types of shock, e.g. the at least three types of shock, may include "unknown", which can act as a catch all for any unidentifiable types of shock.

The method may further comprise a step of processing the predictive indicator to generate one or more recommended actions for the clinician to respond to the most probable type of shock. This approach provides a clinician with useful information for clinically treating the subject based on the most probable type of shock. This provides an improved clinical decision support system.

In some examples, the method comprises generating, for each numeric value, a confidence measure representing a confidence of the numeric value. A confidence measure can help a clinician to understand the model's prediction and its certainty. This information can prove valuable in assessing the condition of the subject, e.g. to establish whether or not the predicted type of shock is actively exhibited by the subject.

The method may further comprise providing, at an output interface, a user-perceptible output of the predictive indicator and optionally the numeric values. Of course, any other form of data generated by the computer-implemented may also or otherwise be provided in the form of a user-perceptible output, including (if generated) the recommended action(s) and/or the confidence measures.

There is also proposed a computer-implemented method comprising: receiving input data from a user interface, the input data indicating whether or not the clinician suspects a subject is exhibiting shock and/or hemodynamic instability; and responsive to the input data indicating that the clinician suspects the subject is exhibiting shock and/or hemodynamic instability, performing any previously described method. In this way, a clinician is able to (manually) trigger the shock type identifying mechanism proposed in this disclosure, e.g. if they suspect the occurrence of shock. This provides a more flexible and user-responsive prediction system.

There is also proposed a computer-implemented method of processing subject data, the computer-implemented method comprising: obtaining subject data comprising one or more data elements, each representative of medical information of the subject; processing the subject data using a hemodynamic analysis method to generate a hemodynamic indicator predicting whether or not the subject is exhibiting hemodynamic instability; and responsive to the hemodynamic indictor predicting that the subject is exhibiting hemodynamic instability, performing any previously described method for predicting a probable type of shock, amongst a plurality of potential types of shock, exhibited by a subject.

This embodiment recognizes that there is a connection between hemodynamic instability and likelihood of shock. By only predicting the type of shock when a hemodynamic instability is predicted, an accuracy of a prediction that a subject is exhibiting shock (and therefore, a particular type of shock) is increased. In particular, this approach reduces the chances that a type of shock will be predicted for a subject when the subject is not exhibiting any shock whatsoever.

This embodiment is particularly advantageous when the types of shock include (e.g. exclusively) cardiogenic shock, septic shock, and hypovolemic shock. These types of shock have been specifically identified as resulting from hemodynamic instability, such that identifying hemodynamic instability as a condition for performing a shock prediction method increases the accuracy of identifying that a subject exhibits shock and the particular type of shock.

There is also proposed a computer program product comprising computer program code means which, when executed on a computing device having a processing system, cause the processing system to perform all of the steps of any herein described method.

There is also proposed a processing system configured to predict a probable type of shock, amongst a plurality of potential types of shock, exhibited by a subject. The processing system is configured to: obtain, at an input interface, subject data comprising one or more data elements, each representative of medical information of the subject; for each of the plurality of potential types of shock: process the subject data, using a respective one-versus-rest model, to produce respective numeric values, wherein each respective numeric value indicates a likelihood of whether the subject is exhibiting a respective type of shock over any other type of shock in the plurality of potential types of shock; and process the numeric values using a classification model to generate a predictive indicator indicating the most probable type of shock amongst the plurality of potential types of shock.

In some examples, the classification model is configured to: perform a logistic function on each numeric value to produce a numeric probability for each numeric value, each numeric probability indicating a probability on a predetermined numeric scale that the subject exhibits a respective type of shock; and generating a predictive indicator identifying the type of shock associated with the numeric probability having the greatest value.

In other examples, the classification model comprises a machine-learning model trained to receive, as input, at least the numeric values and provide, as output, the predictive indicator.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
- Figure 1: illustrates a mechanism for use in an embodiment;
- Figure 2: illustrates an analytics suits for use in an embodiment; and
- Figure 3: is a flowchart illustrating a method according to an embodiment.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism or model for shock type classification that is able to differentiate between different types of shock, e.g. among patients with suspected hemodynamic instability. Respective one-versus-rest models are used to generate a numeric value for each of a plurality of shock types, each numeric value indicating a predicted probability that a subject exhibits that particular shock type over other types. A classification process is then performed to select the most likely shock type based on the numeric values.

Embodiments of the invention can be employed in any suitable clinical decision support or analytics suite/environment, such as those used in patient care systems. Example patient care systems include bedside monitors, hospital-specific information systems, and electronic medical record systems.

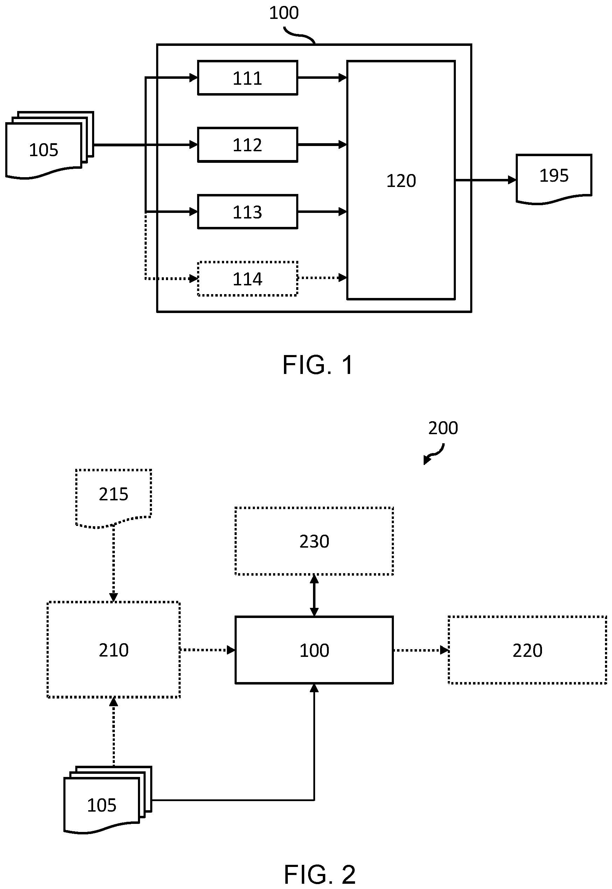

Figure 1 illustrates an approach adopted by embodiments, illustrating a shock type classification mechanism 100. The shock type classification mechanism 100 can be employed to predict a probable type of shock, amongst a plurality of potential types of shock, exhibited by a subject.

The shock type classification mechanism 100 schematically illustrates an approach that can be adopted by a method or system according to various embodiments. Thus, each block of the mechanism 100 may represent a process/step performed by a (computer-implemented) method or by a respective module of a processing system or a data structure used therein.

The shock type classification mechanism 100 employs a system of one-versus-rest models 111, 112, 113, 114. Each one-versus-rest model predicts whether the subject exhibits one particular type of shock over the others by processing subject data 105. In this way, each one-versus-rest model is designed or configured for predicting the occurrence of a particular type of shock over other types. Suitable examples of shock that a one-versus-rest model may be designed for identifying include cardiogenic, septic, and hypovolemic shock. Other types of shock include anaphylactic shock and neurogenic shock.

In some examples, a one-versus-rest model is configured to predict an occurrence of an unknown shock. In the context of this disclosure, an unknown shock is a shock that does not fall into a category covered by any of the other one-versus-rest models. This can effectively act as a catch all for any types of shock not covered by the other one-versus-rest models.

In preferred examples, the one-versus-rest models comprise at least three one-versus-rest models, configured to detect a respective one of cardiogenic, septic, and hypovolemic shock. Thus, there may be a first one-versus-rest model 111 configured to detect cardiogenic shock (over other types of shock); a second one-versus-rest model 112 configured to detect septic shock (over other types of shock); and a third one-versus-rest model 113 configured to detect hypovolemic shock (over other types of shock). An optional fourth one-versus-rest model 114 may be designed for detecting an unknown shock (i.e. a shock that does not fall within the classifications of the other three one-versus-rest models).

The subject data 105 may comprise any suitable information about a subject that can be processed to identify or determine whether the subject is exhibiting a particular type of shock. The subject data 105 may, for instance, comprise information contained in a medical record of the subject and/or information obtained from live monitoring of the subject.

Examples of suitable features to include in subject data include vital signs (e.g. heart rate, respiratory rate, temperature and so on), lab results, high frequency waveforms such as electrocardiograms (ECG). Further examples of suitable subject data include medical images (e.g. derived from bedside cameras or imaging systems) and qualitative observations, e.g. provided by a clinician at an input interface, such as indicators indicating whether or not the subject exhibits: cold extremities, clammy skin and other similar indicators of shock.

Thus, the subject data may comprise information about a subject that is responsive to (i.e. changes in response to) the presence and/or absence of at least one potential type of shock that may be exhibited by a subject.

The subject data 105 may be provided, at least in part, by a storage unit or device. Thus, the shock-type classification mechanism 100 may obtain the subject data 105 from a storage unit or device. In some examples, at least part of the subject data is provided by a user interface (e.g. by a user inputting information about the subject at the user interface). In some examples, at least part of the subject data is provided by a subject monitoring device, that generates (live) subject data about the subject (under investigation).

Each one-versus-rest model 111, 112, 113, 114 is configured to generate a numeric value or measure indicating a likelihood of whether the subject is exhibiting a respective type of shock over any other type of shock in the plurality of potential types of shock. Thus, the numeric value effectively acts as a measure of probability that the subject is exhibiting that particular type of shock over any other type of shock.

In this way, each one-versus-rest model is specifically designed and/or trained for identifying a single particular classification of shock type. In other words, each one-versus-rest model is effectively a (trained) classifier for a particular shock type. To achieve this goal, each one-versus-rest model may have been trained with positive examples of the particular shock type and negative examples of any other shock type, to be able to discriminate between the shock types.

Each one-versus-rest model may be configured to use a subset or subgroup (i.e. not all) of the features contained in the subject data to generate the numeric measures. This approach recognizes that different types of shocks may be characterized by different characteristics or features of subject data. The precise features used by a particular one-versus-rest model may be defined in a training procedure for the said one-versus-rest model.

The numeric values or measures (provided by the plurality of one-versus-rest models) are then processed using a classification model 120. The classification model generates a predictive indicator 195 indicating the most probable type of shock amongst the plurality of potential types of shock.

The predictive indicator may, for instance, contain textual or numeric information identifying the most probable type of shock.

In one example, the classification model is configured to perform a logistic function on each numeric value to produce a numeric probability for each numeric value. Each numeric probability may indicate a probability (e.g. on a predetermined numeric scale) that the subject exhibits a respective type of shock. The classification may then generate a predictive indicator identifying the type of shock associated with the numeric probability having the greatest value.

In this example, the logistic function may be a softmax function or other logistic regression function.

This approach provides a simple and easily adoptable mechanism for identifying the most probable type of shock amongst the plurality of shock types from the outputs of the one-versus-rest models.

In another example, the classification model 120 comprises a machine-learning model. The machine-learning model may trained to receive, as input, at least the numeric values and provide, as output, the predictive indicator 195. Use of a machine-learning model may provide improved accuracy over the simpler logistic function approach, at the expense of requiring additional processing power and complexity.

The machine-learning model may effectively act as a super-classifier, for classifying the type of shock based on the outputs of the one-versus-rest models.

In preferred embodiments, the classification model is configured to further receive, as input, additional subject information containing one or more additional data elements, each representative of medical information of the subject. The additional data elements provide (further) medical information that can be used to more accurately identify which of the shock types the subject is most likely to be exhibiting.

The additional data elements may contain some or all of the subject data. The additional data elements may contain further information about the subject not contained in the subject data, i.e. "additional subject data". Suitable additional subject data may include, for instance, admission diagnosis and/or comorbidities. Use of the subject (i.e. patient) information by the classification model yields more accurate predictions of the type of shock.

Of course, the shock type classification mechanism 100 may provide, at an output interface, a user-perceptible output of the predictive indicator and optionally the numeric values.

It has been described how one-versus-rest models are used to generate numeric predictors. A one-versus-rest model is a particular type of model or algorithm that discriminates for or identifies a particular classification (here: a type of shock) over other possible classification options (here: other types of shock). A one-versus-rest model may be alternatively called a one-versus-many model.

Examples of one-versus-rest models will be familiar to the person skilled in the art of data processing. Generally, a one-versus-rest model is a trained or taught algorithm that processes some input data to generate a numeric indicator of the probability that the input data is an example of a particular type or class (as opposed to any other type or class).

Thus, each one-versus-rest model may be a machine-learning algorithm that generates a numeric measure of likelihood that a subject exhibits a particular type of shock. The (optional) machine-learning model of the classification model is another example of a machine-learning algorithm.

A machine-learning algorithm is any self-training algorithm that processes input data in order to produce or predict output data. In scenarios in which the machine-learning algorithm is used for a one-versus-rest model, the input data comprises subject data and the output data comprises a particular type of shock. In scenarios in which the machine-learning algorithm is used for a classification model, the input data comprises at least the numeric values (output by one-versus-rest models) and the output data comprises an identification of the most likely type of shock.

Suitable machine-learning algorithms for being employed in the present invention will be apparent to the skilled person. Examples of suitable machine-learning algorithms include artificial neural networks, logistic regression, support vector machines or Naive Bayesian models are suitable alternatives.

One particularly suitable example is an artificial neural network (or, simply, neural network), which is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error, e.g. calculated by an appropriately configured loss function, between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

For training a one-versus-rest model specifically for use in this disclosure, the training input data entries correspond to example subject data, and the training output data entries correspond to a numeric indicator of the classification of the specific shock type. The training input and output data entries should include examples of both subjects exhibiting that specific type of shock, and subjects exhibiting other types of shock (to facilitate accurate discrimination of the one-versus-rest model).

For the one-versus-rest models, the training dataset could be generated by first curating a dataset of patients with all shock types to be predicted, i.e. all of the shock types that are to be predicted by the one-versus-rest models. The patients may include only those patients having a diagnosed shock type (e.g. from clinical analysis or patients).

Suitable training sets for the machine-learning model of the classification model could be readily prepared by the person skilled in the art.

Figure 2 illustrates various approaches and options that make use of the previous described shock type classification mechanism 100. Thus, more generally, Figure 2 illustrates a hemodynamic analysis suite 200 that employs a shock type classification mechanism 100 and at least one other optional module (which are hereafter described).

As before, the hemodynamic analysis suite 200 schematically illustrates an approach that can be adopted by a method or system according to various embodiments. Thus, each block of the hemodynamic analysis suite 200 may represent a process/step performed by a (computer-implemented) method or by a respective module of a processing system or a data structure used therein.

The hemodynamic analysis suite 200 may comprise an initiation module 210, which initiates the actions of the shock type classification mechanism 100.

In some examples, the shock type classification mechanism 100 may (only) be initiated, i.e. start processing subject data to provide an output, responsive to receiving an indication that the subject (under investigation) is predicted to be exhibiting shock - i.e. when it is though that the subject is showing signs of shock.

The shock type classification mechanism 100 may be initiated, for example, responsive to a user input 215 (e.g. at a user interface). The user input may indicate whether or not a clinician suspects or believes the subject is showing signs/symptoms of shock. In this way, a clinician is able to indicate a suspicion of shock, and initiate the shock type classification mechanism.

Thus, the initiation module 210 may be a processing module configured to receive a user input 215, determine whether the user input indicates a clinician's suspicion of shock and, responsive to (i.e. only if) the user input indicating a suspicion of shock, initiating the shock type classification mechanism 100.

In another embodiment, the shock type classification mechanism 100 may be initiated responsive to an automatic prediction of the existence of shock or another indicator that preludes or accompanies shock. For instance, the shock type classification mechanism may be responsive to a hemodynamic stability index (HSI), e.g. generated by a hemodynamic analysis suite. In particular examples, the shock type classification mechanism 100 may only be initiated responsive to an HSI indicating instability.

A hemodynamic stability index (HSI) is an indicator (such as score, measure or binary indicator) that indicates whether or not the subject is predicted to have stable or unstable blood flow. Hemodynamic instability is a key indicator of shock, such that detecting hemodynamic instability effectively performs a task of detecting probable shock.

Various approaches for determining or generating a hemodynamic stability index are well known to the skilled person. For instance, US 2014/323846 describes a system for determining a hemodynamic status of an individual through a blood pressure related index. US 2015/145691 describes a method of rendering hemodynamic instability index indicator information. EP3416542B1 describes another method for generating a hemodynamic instability risk score. Any of these, or other, approaches could be used to determine an HSI.

Thus, the initiation module 210 may be a processing module configured to receive subject data 105, generate a hemodynamic stability index (HSI) by processing the subject data and, responsive to (i.e. only if) the generated HSI indicating/indicates instability, initiating the shock type classification mechanism 100. Appropriate methods for generating an HSI have been previously described.

The initiation module 210 may be configured to provide, at a user output interface, a user-perceptible output (e.g. a visual representation or audible representation) of the generated HSI.

The use of a shock type classification system 100 can thereby provide a valuable function or extension for existing hemodynamic analytics suites 200, by facilitating the identification of further information about the type of shock (whereas an HSI may only indicate whether or not there is a probable shock). This facilitates the provision of valuable clinical information for aiding a clinician, and thereby provides an improved clinical decision support system.

If the hemodynamic analytics suite 200 contains a module for generating a HSI (such as that previously described for use in the initiation module), then the subject data processed by each one-versus-rest model may contain the generated HSI.

In some examples, the output of the shock type classification mechanism 100 (the predictive indicator 195 of the type of shock) may be processed to determine or identify recommendations for possible actions, including tests and treatments, for addressing the shock.

Thus, the hemodynamic analysis suite 200 may comprise a recommendation module 220 configured to generate one or more recommendations for possible actions by processing the identified type of shock. The recommendation module 220 may be configured to provide, at a user output interface, a user-perceptible output (e.g. a visual representation or audible representation) of the identified recommendations.

Identifying recommendations may be performed, for instance, using a simple look-up table to correlate the type of shock to one or more recommended actions.

In another example, the type of shock is processed together with subject information (e.g. part or all of the subject data and/or additional information about the subject) to determine the recommendations. The processing may be performed by an appropriately trained machine-learning method, using a look-up table or other task recommendation techniques. For instance, some embodiments may employ a clustering algorithm to identify, amongst a group of historic subjects who exhibited a same type of shock as the identified shock, similar historic subjects to a current subject (based on information about the current subject), and identify recommendations based on historic actions taken for any similar historic subjects.

In some embodiments, the shock type classification mechanism 100 is further configured to generate, for each numeric value, a confidence measure representing a confidence of the numeric value. Thus, each one-versus-rest model may further generate a confidence measure in addition to the numeric value. Approaches for generating a confidence value would be readily apparent to the person skilled in the art and familiar with one-versus-rest models. For instance, a confidence measure can be computed through bootstrapping.

A confidence measure aids in understanding the accuracy, certainty and reliability of a prediction. This information thereby provides valuable information for helping in assessing or analysising the subject, e.g. to persuade a clinician to independently establish whether or not the predicted type of shock is exhibited by the subject and reduce over-reliance upon an automatically generated (and potentially inaccurate) indicator.

In some examples, the output of the shock type classification system is further enhanced by generating one or more feature indicators, which each indicate a feature of the subject data that contributed to one of the numeric measures. The one or more feature indicators may only be generated for the shock type that was identified by the classification model. In particular, the one or more feature indicators may indicate a value of each feature that contributed to one of the numeric measures.

The one or more feature indicators may be generated by: for the numeric measure, identifying one or more features of the subject data based on the type of shock identified by the numeric measure; and, for each identified feature, generating a respective feature indicator indicating a value or measure of the identified feature in the subject data.

This embodiment recognizes that different shock types are associated with different subject data features. For example, a hypovolemic shock prediction is often associated with significantly lower level of hemoglobin, a septic shock prediction is associated with higher level of lactate. Obtaining values for the specific features can thereby provide interpretive information for helping clinicians understand the model prediction.

In other examples, for a particular one-versus-rest model, a class activation mapping (CAM) approach may be used to identify any features of the subject data that most heavily influence the output of the one-versus-rest model. The values of any identified features may then be provided as the feature indicators.

As yet another example, a feature indicator may be generated by determining a feature importance score for features of the subject data. A feature having an importance score greater than some predetermined threshold may be identified in a feature indicator. In other examples, the feature importance scores are themselves used as feature indicators. Feature importance scores can be computed using the Shapley additive explanations.

As previously mentioned, the one or more feature indicators may only be generated for the shock type that was identified by the classification model. Thus, the predictive indicator indicating the most probable type of shock amongst the plurality of potential types of shock may be used to identify the most probable type of shock, and one or more feature indicators may be generated for the numeric measure of the one-versus-rest model associated with that type of shock. This reduces a chance of confusion of the clinician as to the relevance of the feature indicators, e.g. avoids miscommunication with feature indicators generated for other types of shock that were not identified by the classification model.

The one or more feature indicators may be generated by the shock type classification mechanism 100 itself, or by a separate feature identification module 230 that is able to communicatively interact with the shock type classification mechanism 100, and the one-versus-many models in particular.

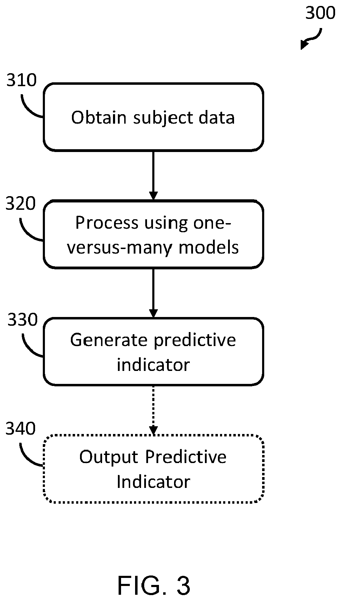

Figure 3 illustrates a method 300 according to an embodiment of the invention. The method is configured to execute or adopt any above-described shock type classification mechanism, and may be further adapted to perform any further extensions for the mechanism (such as those described with reference to Figure 2).

Thus, the method 300 is configured to predict a probable type of shock, amongst a plurality of potential types of shock, exhibited by a subject. The following described steps provide an overview of the fundamental underlying actions of the method.

The method comprises a step 310 of obtaining subject data comprising one or more data elements, each representative of medical information of the subject.

The method further comprises a process 320 of for each of the plurality of potential types of shock: processing the subject data, using a respective one-versus-rest model, to produce respective numeric values, wherein each respective numeric value indicates a likelihood of whether the subject is exhibiting a respective type of shock over any other type of shock in the plurality of potential types of shock.

The method 300 also comprises a step 330 of processing the numeric values using a classification model to generate a predictive indicator indicating the most probable type of shock amongst the plurality of potential types of shock.

The method 300 may further comprise a step 340 of providing, at an output interface, a user-perceptible output of the predictive indicator and optionally the numeric values. Of course, the method may be configured to provide a user-perceptible output of any other value, indicator or parameter generated by the method (e.g. if appropriately adapted to perform the act of any herein described shock-type classification mechanism or additional feature) may be provided at an/the output interface.

The skilled person would be readily capable of developing a processing system for carrying out any herein described method. Thus, each step of the flow chart may represent a different action performed by a processing system, and may be performed by a respective module of the processing system.

For instance, a step of obtaining may be performed by an input interface of a processing system. Processing steps may be performed by a processing unit or (micro)processor of a processing system.

Embodiments may therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method. The computer program may be stored on a non-transitory storage medium, such that there is also proposed a non-transitory storage medium containing a herein described computer program.

In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A computer-implemented method (300) of predicting a probable type of shock, amongst a plurality of potential types of shock, exhibited by a subject, the computer-implemented method comprising:
obtaining (310) subject data (105) comprising one or more data elements, each representative of medical information of the subject;
for each of the plurality of potential types of shock:
processing (320) the subject data (105), using a respective one-versus-rest model (111, 112, 113, 114), to produce respective numeric values, wherein each respective numeric value indicates a likelihood of whether the subject is exhibiting a respective type of shock over any other type of shock in the plurality of potential types of shock; and
processing (330) the numeric values using a classification model (120) to generate a predictive indicator (195) indicating the most probable type of shock amongst the plurality of potential types of shock.

2. The computer-implemented method of claim 1, wherein the classification model is configured to:
perform a logistic function on each numeric value to produce a numeric probability for each numeric value, each numeric probability indicating a probability on a predetermined numeric scale that the subject exhibits a respective type of shock; and
generate a predictive indicator identifying the type of shock associated with the numeric probability having the greatest value.

3. The computer-implemented method of claim 1, wherein the classification model comprises a machine-learning model trained to receive, as input, at least the numeric values and provide, as output, the predictive indicator.

4. The computer-implemented method of claim 3, wherein the classification model is configured to further receive, as input, additional subject information containing one or more additional data elements, each representative of medical information of the subject.

5. The computer-implemented method of any of claims 1 to 4, wherein the plurality of potential types of shock includes at least three types of shock.

6. The computer-implemented method of claim 5, wherein the at least three types of shock comprises: cardiogenic shock, septic shock, and hypovolemic shock.

7. The computer-implemented method of any of claims 1 to 6, further comprising processing the predictive indicator to generate one or more recommended actions for the clinician to respond to the most probable type of shock.

8. The computer-implemented method of any of claims 1 to 7, further comprising generating, for each numeric value, a confidence measure representing a confidence of the numeric value.

9. The computer-implemented method of any of claims 1 to 8, further comprising providing, at an output interface, a user-perceptible output of the predictive indicator and optionally the numeric values.

10. A computer-implemented method comprising:
receiving input data from a user interface, the input data indicating whether or not the clinician suspects a subject is exhibiting shock and/or hemodynamic instability; and
responsive to the input data indicating that the clinician suspects the subject is exhibiting shock and/or hemodynamic instability, performing the method of any of claims 1 to 9.

11. A computer-implemented method of processing subject data, the computer-implemented method comprising:
obtaining subject data comprising one or more data elements, each representative of medical information of the subject;
processing the subject data using a hemodynamic analysis method to generate a hemodynamic indicator predicting whether or not the subject is exhibiting hemodynamic instability; and
responsive to the hemodynamic indictor predicting that the subject is exhibiting hemodynamic instability, performing the method of any of claims 1 to 9.

12. A computer program product comprising computer program code means which, when executed on a computing device having a processing system, cause the processing system to perform all of the steps of the method according to any of claims 1 to 11.

13. A processing system configured to predict a probable type of shock, amongst a plurality of potential types of shock, exhibited by a subject, the processing system being configured to:
obtain (310), at an input interface, subject data (105) comprising one or more data elements, each representative of medical information of the subject;
for each of the plurality of potential types of shock:
process (320) the subject data, using a respective one-versus-rest model (111, 112, 113, 114), to produce respective numeric values, wherein each respective numeric value indicates a likelihood of whether the subject is exhibiting a respective type of shock over any other type of shock in the plurality of potential types of shock; and
process (330) the numeric values using a classification model to generate a predictive indicator (195) indicating the most probable type of shock amongst the plurality of potential types of shock.

14. The processing system of claim 13, wherein the classification model is configured to:
perform a logistic function on each numeric value to produce a numeric probability for each numeric value, each numeric probability indicating a probability on a predetermined numeric scale that the subject exhibits a respective type of shock; and
generating a predictive indicator identifying the type of shock associated with the numeric probability having the greatest value.

15. The processing system of claim 13, wherein the classification model comprises a machine-learning model trained to receive, as input, at least the numeric values and provide, as output, the predictive indicator.
